# EUROPEAN PATENT APPLICATION

(11) **EP 2 243 839 A1**
(43) Date of publication of application: **27.10.2010**
(21) Application number: 10160485.8
(22) Date of filing: 20.04.2010
(51) Int. Cl.: C12P 7/06

(54) **Ethanol production unit and method for the production of ethanol**

(30) Priority: 21.04.2009 NL 2002772
(71) Applicant: Tapergie International B.V., 1251 GN Laren (NL)
(72) Inventor: Smits, Willie, 2517 EA, Den Haag (NL)
(74) Representative: Langenhuijsen, Bastiaan Wilhelmus Herman

(57) **Abstract**

The invention relates to an ethanol production unit, comprising at least one collecting tank provided with intake means (4) for taking in an aqueous sugar solution, preferably palm juice, a fermentor (9) connected to the collecting tank, for converting the aqueous sugar solution supplied from the collecting tank to a fermented mixture comprising water and ethanol, at least one ethanol concentrator (13) connected to the fermentor, provided with at least one outlet for concentrated ethanol obtained from the fermented mixture, energy generating means for providing energy for operating the fermentor and/or the ethanol concentrator, wherein the intake means are provided with quality control means for determining the amount of sugar in the sugar solution, as well as a method for producing ethanol.

## Description

The invention is related to a Ethanol production unit and a method for the production of ethanol.

Traditional ways of cooking consume relatively high amounts of biomass. This is an increasing problem in developing countries, where forest may disappear as a result of commercial forestry combined with local use of wood for cooking. In some areas, this threatens the continuity of society.

It is an aim of the invention to provide a an additional source of fuel in a cost-effective way.

The invention provides an ethanol production unit, comprising at least one collecting tank provided with intake means for taking in an aqueous sugar solution, preferably palm juice, a fermentor connected to the collecting tank, for converting the aqueous sugar solution supplied from the collecting tank to a fermented mixture comprising water and ethanol, at least one ethanol concentrator connected to the fermentor, provided with at least one outlet for concentrated ethanol obtained from the fermented mixture, energy generating means for providing energy for operating the fermentor and/or the ethanol concentrator, wherein the intake means are provided with quality control means for determining the amount of sugar in the sugar solution. The ethanol production unit provides a decentralised production facility that may be used at a local level to produce ethanol, which may be used as a fuel or fuel additive instead of traditional fuels such as wood. The intake means allow for local producers of aqueous sugar solution to contribute to the production plant. The quality control means for determining the amount of sugar solution allow to record the contribution of each local producer. The contribution to the ethanol production plant may be compensated by for instance a share in the ethanol production of the plant. An increasing local energy demand can be met with a decentralized ethanol production unit, and overproduction of the aqueous sugar solution may become a valuable resource to trade.

The quality control means are preferably means to determine the sugar concentration and the volume of the solution provided by an individual local producer, optionally supplemented with means to determine if certain impurities are present that may hamper the fermentation process. Preferably, the quality control means comprise means to control the pH of the sugar solution, preferably an electronic pH meter. The quality control means may also be employed to determine if a predetermined minimal concentration of sugar in the aqueous sugar solution is met.

Preferably the sugar solutions used as a feed for the fermentor are obtained from a local source, in particular plants rich in sugar. A preferred source is the sugar palm (*Arenga* and its subspecies), which provides a steady production of palm juice as an aqueous sugar solution having a relatively high sugar content. In addition to that, the sugar palm has relatively low requirements for survival and may be grown on difficult terrain not suitable for most typical crops for consumption. The sugar solutions obtained from natural sources typically also include other ingredients, for instance minerals or proteins.

The fermentor is typically an anaerobic fermentor as known in the art. Typically, yeast species are used to perform the fermentation process. In order to enable an efficient fermentation process, the yeast or other microbiological species used for fermentation needs to be provided with optimal environmental circumstances, in particular the presence of nutrients, pH control, temperature control and a water lock to maintain anaerobic conditions and avoid the intrusion of unwanted bacteria or fungus. When using palm juice as a feed stock, the sugar concentration in this feedstock usually needs to be increased in order to obtain a relatively high ethanol production rate.

The energy generating means may be of known types for the generation of heat and/or electricity, depending on the needs of the other parts of the ethanol production unit.

Preferably, the quality control means comprise a refractive index meter. A refractive index meter or refractometer provides a quick and reliable method to determine the composition of a known type of solution (for instance palm juice), once a standard relationship between the measured value and sugar content has been established.

It is also advantageous if the quality control means comprise pH measurement means. The pH may be an indication of bacterial pollution. If a pH in a certain batch is outside predetermined pH limits, the particular jerry can is to be rejected in case of off-spec material as it may pollute the whole feed container. For the fermentation process, the pH of the feed stock needs to be within certain limits to yield an optimum in the fermentation process (dependent on the type of yeast used). Means for adjusting the pH, such as adding acid or base, is preferably also included as a means of control for the fermentation process.

In a preferred embodiment, the intake means are constructed for taking in aqueous sugar solution from a container, preferably a hand-held container. Such containers, for instance a 25 litre jerry can, are particularly suitable for storing and transporting locally produced sugar solutions.

It is advantageous if the intake means are also provided with means to determine the amount of aqueous sugar solution in the container. Combined with the sugar concentration, this offers a rapid means for determine the actual amount of sugar in a given batch of sugar solution. Preferred means are relatively simple mechanic means, in particular weight-based means or physical measuring means such as a level rod for measuring the volume in a standard container, or a scale indicated on a container.

It is preferred if the intake means comprise data reading means for reading an identification tag of the container. Thus, it is relatively easy to identify and keep track of the contribution of a local producer of sugar solution to the plant. The identification tag may be a printed code or card, but is preferably a machine-readable tag such as a personalised bar code or RFID tag that may be incorporated in the container. The data reading means are preferably connected to a processing unit such as a personal computer.

It is preferred if the intake means are provided with recording means for recording the identification tag of a container, the sugar concentration and the amount of aqueous sugar solution in the identified container. The recording means may for instance be automated recording means such as an automated weight meter or solution level meter, but may also be a personal computer having a keyboard serving to record the data manually.

In a preferred embodiment the recording means are programmed to award a predetermined amount of credit to a person associated with the identification tag, as a function of the amount of sugar taken in as determined from the determined sugar concentration and the amount of aqueous sugar solution. Thus, the amount of sugar contributed to the plant by a local producer is actually converted into credit, which may be used by the producer, for instance as a monetary reward, credit in a savings account or for obtaining an equivalent share of the production of the plant.

Optionally, the fermentor is connected to the collecting tank via an evaporator for concentrating the sugar solution to a predetermined minimal value. The aqueous sugar solution as delivered is preferably already preconcentrated by the local producer, a central thickener unit is energetically favourable for thickening larger amounts of collected aqueous sugar solution.

In a preferred embodiment, the evaporator also comprises water separating means for collecting water from the sugar solution. As concentrating implies the removal of water from the palm juice or other sugar solution, as a result water of a relatively high quality is produced by the evaporator, which may be used for further processing. For instance, the water separating means may be connected to a collecting tank and/or water purification means. Water purification means preferably comprise the physical separation of solid particles and biological treatment of the water in a heliophyte basin, to produce industry-grade or even consumption-grade water.

Preferably, the fermentor comprises at least two, preferably at least three, independently operable batch reactors. As the fermentation process may last for one day or more, having multiple batch operators allows for running several fermentation processes at various stages of the fermentation process. This allows for a continuous production of ethanol by the plant as a whole.

In a preferred embodiment, the ethanol concentrator comprises distillation means. Distillation is a reliable method for concentrating ethanol in an aqueous solution while purifying the mixture of water and ethanol at the same time and removing unwanted components, for instance minerals, which remain as a non-volatile fraction. Advantageously, the outlet for concentrated ethanol is connected to energy generating means for using at least part of the concentrated ethanol of generating energy. The energy from the ethanol may contribute to the operation of the unit, in particular the energy generating means. The energy generating means may for instance comprise the combustion of organic matter such as dried material from palm trees. Combustion of organic materials may be used directly to heat water and generate steam, which may be employed for the generation of electricity in a steam generator.

It is preferred if the unit is provided with a dock suitable for docking at least one animal driven cart, located adjacent to the intake means. Thus, tappers and other local producers may comfortably deliver their produced aqueous sugar solution to the intake means. Animal driven carts are an economic transport solution and environmentally friendly, and may include oxcarts, donkey carts etc. Preferably, the docking station is constructed as a drive-through station. It is advantageous if the docking station is also provided with animal feeding means. Yeast residue from the fermentation process may advantageously be used as a feed stock supplement for the animals used in delivering the aqueous sugar solution.

In an advantageous embodiment, the dock is provided with faeces collecting means. The collected faeces may be used in the energy generating means of the ethanol production unit, for instance in a biogas gasifying unit.

In a preferred embodiment, the ethanol production unit according to the invention is built from connectable modules, wherein each module is integrated in a transportable container. Thus, the ethanol production unit may be manufactured in modules at a suitable location, and may relatively easily be shipped in parts to a remote area. The ethanol production may be assembled rapidly by connecting the modules. Preferably, the containers comply with the sizes and requirements of standard cargo containers, so the plant may be shipped by regular standardized transport vessels such as container ships, trains and trucks.

The invention also provides a method for the production of ethanol, comprising the following steps: collecting batches of aqueous sugar solution, determining the amount of sugar in the aqueous sugar solution, concentrating the aqueous sugar solution to a predetermined minimal concentration of sugar, adjusting the pH to a predetermined value, fermenting the aqueous sugar solution to a fermented mixture comprising water and ethanol, and concentrating the mixture to a mixture having a higher concentration of ethanol. Thus, ethanol may be produced from relatively small local sources, which may be further used as a fuel at a local level, whereas any palm juice overproduction may be used for trading. After collecting the solution, a major part will be used for concentrating the aqueous sugar solution to a predetermined higher concentration of sugar, for instance 20. Adjusting the pH to a predetermined value depends on the type of yeast used. Typically, the predetermined value for the pH is in the range of 4,5-5. For starting the fermentation yeast usually needs to be activated. This may for instance be done by aging yeast in an oxygen rich environment before dosing the yeast into the fermentation reactor liquid feed.

The invention will now be further elucidated by the following non-limiting examples.
Figure 1 schematically describes the ethanol production system according to the invention.
Figure 2 shows a schematic representation of the intake procedure.

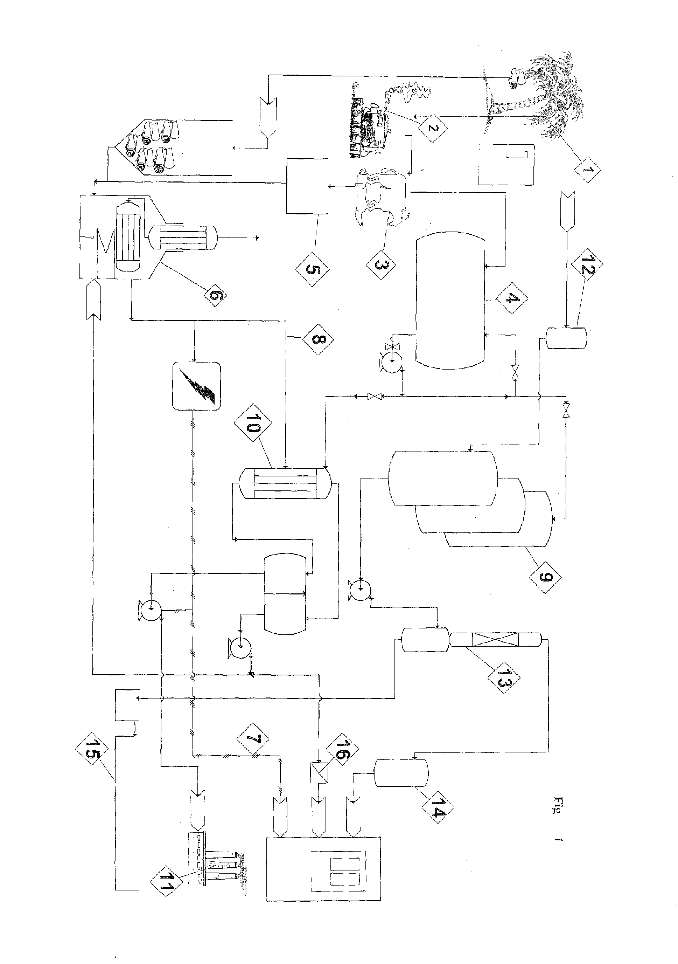

Figure 1 schematically describes the ethanol production system 1 according to the invention. An aqueous sugar-containing solution is collected from a natural source, for instance palm trees 2 which produce palm juice with a sugar content of for instance 11 % Brix. Brix is a measurement indicating the amount of sugar (in particular saccharose) in an aqueous solution, which may for instance be determined by a refractometer or by determining the density of the solution by gravimetry. The sugar concentration of the collected palm juice may be increased by the evaporation of water, for instance to 20% brix, which may be done locally using heating means 2 fuelled by for instance biomass. The locally produced aqueous sugar solution is then transported to the ethanol plant 1, for instance using an oxcart 3. In order to prevent sugar crystallisation from the solution, the sugar solution is taken in preferably at an elevated temperature, for instance 70 °C, at the intake station 4, where sugar solutions from various local suppliers are collected in a tank.

The intake station 4 may be provided with collecting means 5 for faeces. The biogas obtainable from faeces may be used in generating energy, for instance in a biomass- fuelled generator 6. The shown generator 6 produces both electricity 7 as well as heat 8 which is transported as steam, for operating the ethanol plant 1.

The collected sugar solution may be fed to a fermentor unit 9, or to a thickener unit 10 for further concentrating the sugar solution. The thickened solution may have a concentration of for instance 65% Brix. A solution with such a very high sugar concentration has lower transportation costs and a superior resistance towards microbiological degradation and may be temporarily stored or transported over longer distances, for instance for use in a further central processing unit (CPU) 11, for instance for making sugar products.

The fermentor 9 uses yeast 12 in an anaerobic process converting sugar to ethanol and carbon dioxide. Preferably, the fermentor 9 comprises multiple independent batch reactors, which enables virtually continuous production of the plant as a whole, even if part of the batch reactors is out of production due to maintenance or refilling a fresh batch. The collected ethanol may be purified and concentrated to a concentration suitable for use as a fuel using distillation in the distillation unit 13. As a secondary product of distillation, contaminated water is produced. This water still contains organic substances, and must be further purified using for instance a heliophyte filter 15 and/or a drinking water filter 16 in order to obtain water usable in industrial or agricultural processes, or even as drinking water. The distilled ethanol may be collected in a storage tank 14, which may be used to distribute the produced alcohol amongst the contributors supplying sugar water to the plant 1.

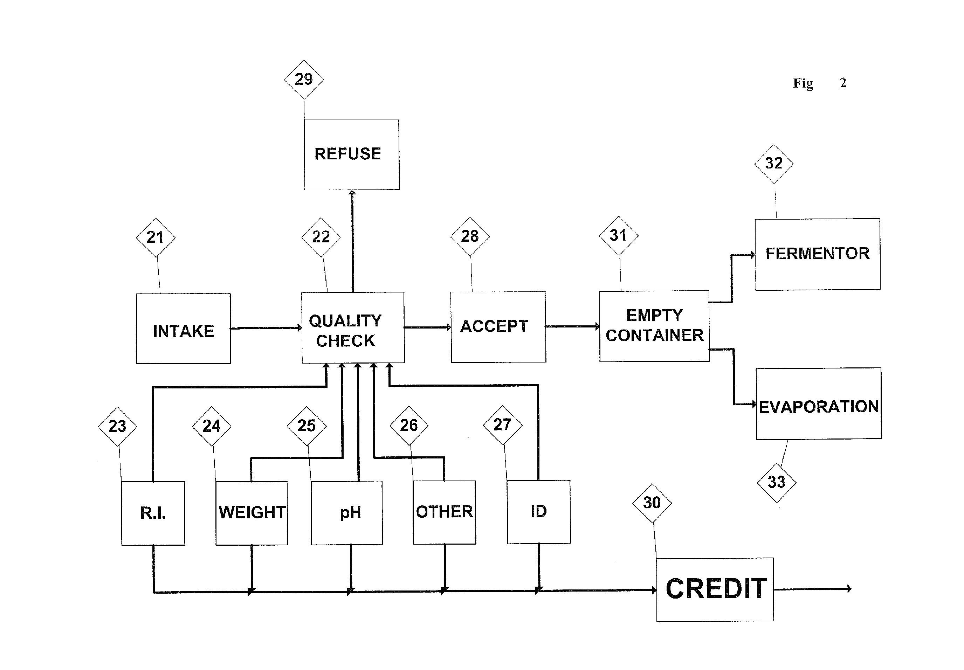

Figure 2 shows a schematic representation of the intake procedure 20 according to the invention. After the intake 21 of a container, for instance a jerry can, of sugar-containing solution, a quality check 22 is performed. This may for instance involve the taking of a refractive index 23 by a refractometer, and the determination of the volume 24, either by determining weight or directly measuring the volume. Also, the supplier of the batch of is identified 25, for instance by an identification code on the supplied container. Also other properties of the batch may be checked 26, for instance the presence of certain contaminants. The quality check 22 results in either acceptance 28 or refusal 29 of the batch, depending on predetermined parameters to be met. If the batch is accepted, the measured sugar concentration and volume may be used to calculate a credit 30 for the supplier, which may for instance be used to obtain a certain amount of ethanol from the plant. The accepted batch 28 further enters the process. The container is emptied 31 into a storage tank or directly into either the fermentor 32 or the thickener unit 33, in order to be processed further.

## Claims

1. Ethanol production unit, comprising
- at least one collecting tank provided with intake means for taking in an aqueous sugar solution, preferably palm juice,
- a fermentor connected to the collecting tank, for converting the aqueous sugar solution supplied from the collecting tank to a fermented mixture comprising water and ethanol,
- at least one ethanol concentrator connected to the fermentor, provided with at least one outlet for concentrated ethanol obtained from the fermented mixture,
- energy generating means for providing energy for operating the fermentor
and/or the ethanol concentrator,
wherein the intake means are provided with quality control means for determining the amount of sugar in the sugar solution.

2. Ethanol production unit according to claim 1, **characterized in that** the quality control means comprise a refractive index meter.

3. Ethanol production unit according to claim 1 or 2, **characterized in that** the quality control means comprise pH measurement means.

4. Ethanol production unit according to any of the claims 1-3, **characterized in that** the intake means is constructed for taking in aqueous sugar solution from a container, preferably a hand-held container.

5. Ethanol production unit according to claim 4, **characterized in that** the intake means are also provided with means to determine the amount of aqueous sugar solution in the container.

6. Ethanol production unit according to any of the preceding claims 4-5, **characterized in that**
the intake means comprise data reading means for reading an identification tag of the container.

7. Ethanol production unit according to claim 6, **characterized in that** the intake means are provided with recording means for recording the identification tag of a container, the sugar concentration and the amount of aqueous sugar solution in the identified container.

8. Ethanol production unit according to claim 7, **characterized in that** the recording means are programmed to award a predetermined amount of credit to a person associated with the identification tag, as a function of the amount of sugar taken in as determined from the determined sugar concentration and the amount of aqueous sugar solution.

9. Ethanol production unit according to any of the preceding claims, **characterized in that**
the fermentor is connected to the collecting tank via an evaporator for concentrating the sugar solution to a predetermined minimal value.

10. Ethanol production unit according to claim 8, **characterized in that** the ethanol concentrator also comprises water separating means for collecting water from the sugar solution.

11. Ethanol production unit according to any of the preceding claims, **characterized in that**
the fermentor comprises at least two, preferably at least three, independently operable batch reactors.

12. Ethanol production unit according to any of the preceding claims, **characterized in that**
the ethanol concentrator comprises distillation means.

13. Ethanol production unit from any of the preceding claims, **characterized in that** the unit is provided with a dock suitable for docking at least one animal driven cart, located adjacent to the intake means.

14. Ethanol production unit according to claim 13, **characterized in that** the dock is provided with faeces collecting means.

15. Ethanol production unit according to any of the preceding claims, **characterized in that**
the ethanol production unit is built from connectable modules, wherein each module is integrated in a transportable container.

16. Method for the production of ethanol, comprising the following steps:
- collecting batches of aqueous sugar solution,
- determining the amount of sugar in the aqueous sugar solution
- concentrating the aqueous sugar solution to a predetermined minimal concentration of sugar,
- adjusting the pH to a predetermined value,
- fermenting the aqueous sugar solution to a fermented mixture comprising water and ethanol,
- concentrating the mixture to a mixture having a higher concentration of ethanol.
